# EUROPEAN PATENT SPECIFICATION

(11) **EP 1 549 759 B1**
(45) Date of publication and mention of the grant of the patent: **28.04.2010**
(21) Application number: 03741878.7
(22) Date of filing: 04.06.2003
(51) Int. Cl.: G01N 33/569

(54) **BACTERIAL TEST METHOD BY GLYCATED INTRACELLULAR ENZYMES**
BAKTERIENTESTVERFAHREN MIT BINDUNG EINER INTRAZELLULARER ENZYME
METHODE D'ESSAI BACTERIOLOGIQUE PAR LIAISON D'ENZYMES INTRACELLULAIRES

(30) Priority: 12.06.2002 US 170133
(43) Date of publication of application: 06.07.2005
(73) Proprietor: Siemens Healthcare Diagnostics Inc., Tarrytown, NY 10591 (US)
(72) Inventor: PUGIA, Michael, J., Granger, IN 46530 (US); BASU, Manju, Granger, IN 46530 (US); HATCH, Robert, P., Elkhart, IN 46514 (US); PROFITT, James, A., Goshen, IN 46528 (US)
(74) Representative: Maier, Daniel Oliver
(86) International application number: PCT/US2003/017688
(87) International publication number: WO 2003/106699

(56) References cited:
- WO-A-00/12556
- WO-A-00/37943
- WO-A-02/09674
- WO-A-85/05631
- WO-A-94/24561
- WO-A-95/05455
- WO-A-95/08344
- US-A- 4 753 893
- US-A- 5 225 330
- US-A- 5 225 330
- US-A- 5 750 357
- US-A- 6 020 208
- US-B1- 6 322 788
- ROTH R I: "Hemoglobin enhances the binding of bacterial endotoxin to human endothelial cells." THROMBOSIS AND HAEMOSTASIS. AUG 1996, vol. 76, no. 2, August 1996 (1996-08), pages 258-262, XP008068493 ISSN: 0340-6245
- ROTH ROBERT I ET AL: "Hemoglobin: A newly recognized binding protein for bacterial endotoxins (LPS)" 1994, PROGRESS IN CLINICAL AND BIOLOGICAL RESEARCH; BACTERIAL ENDOTOXINS: BASIC SCIENCE TO ANTI-SEPSIS STRATEGIES WILEY-LISS, INC., 605 THIRD AVENUE, NEW YORK, NEW YORK 10158-0012, USA; WILEY-LISS, LTD., CHICHESTER, ENGLAND SERIES : PROGRESS IN CLINICAL AN , FOURTH INTERNATIONAL CONFERENCE ON ENDOTOXINS; AMSTERDAM, NETHERLANDS; AUGUST 1993 , XP008068461 ISSN: 0-471-02181-4 binding of hemoglobin to LPS from E. coli
- FOSSET M ET AL: "Intestinal alkaline phosphatase. Physical properties and quaternary structure" BIOCHEMISTRY 1974, vol. 13, no. 9, 1974, pages 1783-1788, XP002377067
- ANONYMOUS: "Medical Dictionary: "binding"" INTERNET ARTICLE, [Online] Retrieved from the Internet: URL:http://www.mondofacto.com/facts/dictio nary?query=binding&action=look+it+up> [retrieved on 2009-06-10]

## Description

### Background of the Invention

This invention relates generally to methods for detecting bacteria in fluids, particularly in biological specimens. More specifically, the invention relates to rapid methods for detecting bacteria in urine and other fluids with improved accuracy compared to those currently available. Although analysis of urine is of particular interest, other fluids, such as blood, serum, water, and the like may be analyzed using the methods of the invention.

A rapid test for bacteria is desirable, for example by using dry test strips of the sort now used for various purposes. At present, urine test strips are used to screen samples and rule out those which do not require laboratory assessment. However, the current tests, such as measurement of nitrites and leukocytes, are not capable of rapidly providing accurate results. Often, many false results are obtained, causing unnecessary laboratory followup analyses. About 50% of a hospital laboratory's workload involves urine specimens and about 90% of these specimens are cultured and analyzed for total and gram negative bacteria. However, only about 10% of urine samples which are cultured for detection of bacteria are actually found to test positive. Clearly, an accurate prescreening of urine could greatly reduce the number of samples sent to the laboratory for analysis.

The market penetration of the presently available test strips is not large, in part because the tests produce false positive results, as later determined by laboratory followup analysis. Thus, a test strip which provides rapid and accurate determination of the presence of bacteria would reduce costs and make it possible to treat bacteria in a patient immediately, rather than waiting for laboratory results.

The present inventors were investigating methods by which bacteria could be detected accurately. One potential approach involved finding substances that could bind to bacteria and then be detected and measured so that the amount of bacteria present could be determined. The problem can be stated as follows: How do substances bind to bacteria and which substances exhibit the properties needed for accurate measurements to be made? The binding should be specific to the bacteria. Non-specific binding can obscure the results since it can vary unpredictably and provide inaccurate results.

Antibodies are recognized as having the ability to attach to bacteria and it was believed that if ALP (alkaline phosphatase), which can be used to detect by color development materials to which it is bound, could be attached to another substance capable of attaching itself to bacteria, it would be possible to measure the amount of bacteria present. At first, experiments indicated that the ALP was bound to bacteria in a non-specific manner and therefore it was considered to present a problem to the development of a reliable method of measuring the amount of bacteria present in a sample. Further investigation was directed toward eliminating non-specific binding of ALP so that only the ALP attached to substances which could bind to bacteria would be measured. Surprisingly, it was found that the belief that the ALP was non-specifically bound to bacteria was not correct and that in fact, it did bind to bacteria, leading to the present invention. As will be seen below, ALP is a preferred substance for measuring the amount of bacteria, but other substances can be used, particularly glycopeptides and glycoproteins.

### Related literature and patents

Methods for rapid testing for bacteria are known, but they differ from the method of the present invention. In one method, an immunoassay for detecting lipopolysaccharides from Gram negative bacteria such as E. Coli, Chlamydia or Salmonella uses a lipopolysaccharide binding protein or an antibody having specific binding affinity to the liposaccharide analyte as a first or second binding reagent (see WO 00/60354 and US 5,620,845). In US 5,866,344 other immunoassays are described for detecting polypeptides from cell walls. Proteins can be purified in a method using polysaccharide binding polypeptides and their conjugates (see US 5,962,289; US 5,340,731; and US 5,928,917). In US 5,856,201 detection of proteins using polysaccharide binding proteins and their conjugates is disclosed. The methods described in the above differ from those of the present invention, as will be seen in the discussion of the present invention below.

The methods which are based on liposaccharide antibodies or binding proteins do not provide a measure of the total bacteria present. They also do not use a glycopeptide or glycoprotein to bind to a bacteria cell. The methods based on polypeptides require antibodies to bind to the bacteria cell wall rather than using glycopeptides or glycoproteins. The methods based on polysaccharide binding polypeptides require the fusion of short sequences of polypeptides onto analytes of interest and employ non-glycated polypeptides to bind to a polysaccharide.

Glycoproteins have been shown to bind to various biomolecules. For example, glycoproteins on a fungus cell surface have been shown to bind to host proteins. Also, glycoproteins excreted from epithelial cells have been shown to bind to lipids and the binding of glycoproteins to carbohydrates is well known. All such interactions of glycoproteins are dependent on many factors, such as ionic strength and pH, and the affinity of the individual proteins for the biomolecules. However, the use of glycoproteins in assays for measurement of bacteria content has not been described heretofore.

Glycoprotein receptors have been isolated on human monocyte cells. Two binding proteins extracted from the cell walls of human monocytes have been shown to have an affinity of 9x10⁺⁶ for binding fructosyllysine (lysyl peptides glycated with glucose) with 10,000 active binding sites per cell. These receptor protein sites are thought to belong to the family of RNA-binding proteins and to be involved in the aging process by binding age related proteins such as glycated albumin. However, the prior art on glycoprotein receptors does not teach that receptors on the cell walls could be used for the detection of cells. There is no means provided for signal generation, whether by color particle or enzymatic reaction that can be used as a measure of the count or detection of cells.

Bacteria are known to attach to host tissue, often by adhesion of bacterial cell membrane to extra-cellular matrix proteins of the host. This binding is known to occur through several modes of interaction, by glycoaminoglycans, collagens, proteins and integrins on their surface. Thus, the cell surface, including bacterial cell surfaces, can be visualized as a mosaic of molecules capable of binding to proteins of the host tissues as well as receptor sites of the host.

The interaction between bacterial cells and glycoproteins is known generally, but the binding of specific glycopeptides to a bacterial cell has not been disclosed. Bacterial cell adhesion has been described to extra-cellular matrix proteins such as fibronectin and lamin. This binding was shown to occur between the cell adhesions and glycated groups on the proteins. Similar results have been shown with connective tissue proteins and bacterial cells. Polypeptide and carbohydrate structures of glycoproteins can vary greatly and the chemical structures of glycopeptides and glycoproteins are often unknown, such as those which bind bacterial cells.

Methods for measuring binding of glycoproteins to bacterial cells have been described; however, the measurement of bacteria by glycopeptide or glycoprotein binding has not. More particularly, binding of glycopeptides or glycoproteins which are enzymes or are attached to detection labels has not been disclosed.

The binding of cell walls to alkaline phosphatase (ALP) is known, but at the present time, it is not possible to assign a precise function to any alkaline phosphatase other than the catalysis of the hydrolysis of phosphomonoester. It is known that tissue damage causes a release of these ALP iso-enzymes providing clinical significance.

Certain ALP iso-enzymes are known to be membrane-bound. Intestinal, liver, bone, kidney and placental alkaline phophatase iso-enzymes are examples of enzymes that are known to be membrane bound to cell walls, including dipeptidylpeptidase, aminopeptidases such as alanine aminopeptidase, endopeptidase, gamma-glutamyl transferase, lactase, alpha-D-glucosidases, hydrolases such as glycosidase and 5' nucleotidase. Cell membrane binding for ALP is known to occur through a C-terminal glycan-phosphatidyl-inositol anchor in which the long chain triglycerides of the anchor are incorporated into the lipoprotein membrane. The C-terminal glycan-phosphatidylinositol anchor is absent from the ALP produced by E Coli bacteria and the ALP from E Coli is considered to be a soluble enzyme. Thus, binding of ALP to E Coli in the present invention would have to occur by another mechanism.

ALP has been used in some diagnostic applications. For example, ALP has been used in an immunoassay diagnostic test as a label for the immunoassay; see US 5,225,328. However, it has not been used in a dry phase test without an antibody for detection of bacteria.

The present inventors have discovered that all bacteria cells have the ability to bind certain glycoproteins through multiple binding sites. As a result of this discovery, they have found that such glycoproteins can be used in test strips having the ability to detect all bacteria present with accuracy, as will be seen in the detailed discussion of the invention which follows.

### Summary of the Invention

The invention is defined by claims 1-12.

### Brief Description of the Drawings

FIG. 1 illustrates the results of Example 1
FIG. 2 illustrates additional results of Example 1.
FIG. 3 illustrates the results of Example 4.
FIG. 4 illustrates the results of Example 7.
FIG. 5 illustrates the results of Example 7.
FIG. 6 illustrates the results of Example 8.
FIG. 7 illustrates the results of Example 8.
FIG. 8 illustrates the effect of pH on ALP activity.
FIG. 9 illustrates the effect of pH on ALP activity.
FIG. 10 illustrates the effect of different cations on ALP binding.
FIG. 11 illustrates the effect of different cations on ALP binding.

### Description of the Preferred Embodiments

### Glycoproteins

Glycoproteins are composed of amino acids with peptide linkages and carbohydrates. Glycoproteins can be attached to bacteria through charge attraction and shape to molecules on the cell wall. As will be seen in the examples below, the amount of the glycoprotein bound to bacteria cells will vary depending on several factors, including the molecular structure, presence of metals, ionic strength, and pH of the environment.

Glycoproteins, in which one or more carbohydrate units have been attached covalently to the protein, are intra cellular enzymes (e.g., glutamate dehdrogenase, ALP, lacate dehdrogenase).

The carbohydrate monomer units that are commonly attached to proteins include galactose (Gal), mannose (Man), glucose (Glu), N-acetylglucosamine (GlcNAc), N-acetylgalactosamine (Gal NAc), sialic acids (SA), fucose and xylose. The carbohydrate chains occur with a wide variety of lengths and structures, but some typical structures encountered are Man-GlcNAc-, GalNAc(Gal)(SA)-, Man(Man(Man)₂) (Man(Man))-GlcNAc -GlcNAc-, Man((Man-GlcNAc-Gal-SA)₂₋GlcNAc -GlcNAc- and those listed in Table 2 below.

The carbohydrate chains are generally attached to proteins via the hydroxyl groups of serine (Ser) or threonine (Thr) amino acid residues, the amide N atom of asparagine (Asn) side chains or through hydroxy-lysine (Hyl) residues. The particular Ser and Thr residues O-glycosylated do not appear to occur in unique amino acid sequences, therefore Ser or Thr can be connected to any aminoacid, such as Ser-X, Thr-X, where X can be any amino acid. The glycosylation of Hyl residues occurs in a characteristic sequence -Gly-Y-Hyl-Z-Arg-, where Y and Z are any amino acids. The Asn residues N-glycosylated occur in the sequence of -Asn-X-Ser- or -Asn-X-Thr-, where X may be any of the normal amino acids, other than Pro.

One particularly effective glycoprotein is alkaline phosphatase (ALP). The amount of the glycoprotein will depend upon the amount of the bacteria present in the sample; for example, when bacteria is present, a certain amount of glycoprotein will be dependent on the number of binding sites and strength of the binding constant. With a given glycoprotein and bacteria cell type the binding sites are fixed and the amount of glycoprotein bound is directly proportional to the amount of bacteria present.

### Additional Components

The method of the invention may be applied in dry test strips familiar to those skilled in the art, or in wet test methods such as those described in the examples below. Depending on the specific technique, buffering compounds, substrates for the glycoprotein enzyme amplification compounds, and other additives such as blocking compounds may be present.

It has been discovered that adding specific transition state metals increase protein binding to bacteria cell walls. While not required, the use of specific transition state metals increases the sensitivity of an assay based on glycated protein binding to bacteria.

In a particularly preferred embodiment of the invention, such metals are used to increase the response of the labeling moiety. Various metals have been evaluated. Of these, zinc, copper, and iron have been found to have a beneficial effect, particularly zinc, as will be seen in the examples below.

Substrates for ALP include the phosphate esters of the following organic groups, primary and aliphatic alcohol, sugars, sugar alcohols, phenols, naphthols and nucleosides. Examples of substrates forming visual color include naphthol-AS-BI-phosphate, naphthol-AS-MX-phosphate, p-nitrophenol phosphate phenylphosphate (PPNP), indoxylphosphate, e.g., bromo-chloro-indolyl-phosphate (BCIP), phenolphthalein phosphate, thymolphthalein monophosphate and diphosphate, beta-naphthylphosphate, dicyclohexylammonium salt of PPNP for stability, thymolphthalein monophosphate, phenolphthalein diphosphate, carboxyphenyl phosphate, beta-glycerophosphate and beta-glycerolphosphate. Examples of fluorescent substrates for ALP include methylfluoresceine alpha-naphthyl phosphate. Alkaline phosphatase can be measured by a wide range of chemiluminescent and bioluminescent substrates. Examples of chemiluminescent substrates for ALP include adamantyl 1,2-dioetane aryl phosphate, 5-bromo-4-chloro-3-indolyl phosphate, phenacyl phosphate, NADP, ascorbic acid 2-O-phosphate, cortisol-21-O phosphate, N,N' -dimethyl-9,9' bisacridinium dinitrate, indolyl derivatives, e.g., 5-bromo-4chloro-3-indolyl phosphate disodium salt (BCIP-2Na), D-luciferine-O-phosphate and adamanyl 1,2-dioxetane aryl phosphate (AMPPD).

Various buffers, both non-transphosphorylating and those of varying degrees of transphosphorylating property have been used for ALP determinations (i.e., Carbonate, 2-amino-2-methyl-1-propanol and diethanolamine). Buffers commonly utilized for ALP include ethylaminoethanol (pKa 9.9), diethanolamine (pKa 8.7), tris- (hyroxymethyl) aminomethane (pKa 7.8), 2-amino-2-methyl-1-propanol MAP (pKa 9.3), 2-amino-2-methyl-1,3-propanediol (pKa 8.6), sodium carbonate, sodium bicarbonate (pKa 9.9), glycyl-glycine (pKa 8.2), glycine (pKa 9.6), and barbital (pKa 7.44) with activity measured at pH ranges of 7 to 10.

Additional additives such as enzyme co-factors may be used to enhance the reaction conditions for enzymes. Mannitol and other alcohols can be used to increase ALP substrate rates. In the case of ALP, at least one equivalent of Zn, Ca and Mg metal for each ALP molecule will be present to provide catalytic activity and possibly also for maintenance of the native enzyme structure. Enzyme inhibitors are also often used to modulate enzyme assay ranges and mask interference. In the case of ALP, known inhibitors include cysteine, EDTA and thioglycolic acid , L-phenylalanine, L-homoarginine, L-tryptophane, L-leucine, levamisol and imidazole. It is also known that salts such as sodium chloride can be used to control enzymes. It is also known that surfactants such as sodium dodecyl sulfate and bile acids modulate enzyme assay ranges and sensitivity.

Enzyme amplification systems can also be used to increase detection limits for enzyme assays. Several enzyme amplification methods for the detection of alkaline phosphatase are known. These include the formation of formazan (INT-violet colorimetrically or resazurin fluorimetrically) through enzyme systems (e.g., diaphorase and alcohol deyhydrogenase) that employ NAD co-factor and rely on ALP to dephosphorylate NADP enzyme to produce NAD. For example, nicotinamide adenine dinucleotide phosphate (NADP) conversion to NAD⁺ by ALP has been used for amplification. The NAD⁺ compound was then reduced to NADH by alcohol dehydrogenase in the presence of ethanol included in the reaction medium. In turn, NADH in the presence of diphorase was converted back into NAD with simultaneous reduction of tetrazolium salt also present in the medium. This resulted in an accumulation of colored soluble formazen dye, proportional to the concentration of NAD⁺ generated by AP. The newly formed NAD⁺ is recycled many times, resulting in a 100-fold increase in sensitivity.

Blocking compounds selected from the group consisting of polymers, non-glycated proteins, non-glycated polypeptides, and polysaccharides may be included in order to reduce interference or improve color. Interference is improved by preventing non-specific binding by interfering substances to bacteria by instead binding interfering substances to the blocking compound. Color is improved by acting as a spreading layer which allows color to be uniform in dry reagents.

### Test Methods

The use of glycoproteins for the detection of bacteria can be applied to a variety of test methods. The methods require combining a glycoprotein with sample to be assayed, separating the glycoprotein bound to bacteria from free unbound glycoprotein and measuring bound or free glycoprotein. Such steps can be accomplished through a variety of fluid handling analyzers such as centrifugal, microfluidic devices, chromatography strips, filtration and microplate readers, to name a few.

The effectiveness of glycoproteins for the detection of bacteria is measured in the same way for all test methods. Effectiveness is measured by obtaining a bacteria detection signal that is three standard deviations from the signal obtained in the absence of bacteria.

In order for the glycoproteins to be effective at detecting 1000 bacteria cell/mL, the association constant must be at least 10⁺⁶ and the number of binding sites at least 100. These measures of the bind strength for glycoprotein to bacteria and of the number of binding sites for glycoprotein to bacteria allow a sufficient bacteria detection signal. The 1000 bacteria cell/mL detection limit is the minimal clinically desired threshold. A sufficient background reading for the glycoprotein binding to other specimen components, e.g., other proteins, must be an association constant of less than 10⁺⁴. Using ALP as a representative example, a binding constant of 5x10⁺⁶ and the number of binding sites was estimated to be 590.

### Example 1

### Bacteria assay by binding of Intestinal Alkaline Phosphatase

Bacterial cells (10⁶ to 10⁸ cells/mL) were washed twice with water after centrifugation to separate the cells into a packed pellet from supernatant liquid. The washed cells in pellet form were suspended in 40 µL water and 10 µL of aqueous bovine intestinal alkaline phosphatase (ALP) was added (2 µg or 10,000 Units). The mixture was left at room temperature for 30 minutes and then centrifuged, after which the bacterial pellets were washed with water 4-5 times (50 µL). All the washing supernatants were combined. A blank without cells was diluted in the same way. The final pellets were suspended in 50 µl water and both supernatants and cell suspensions were assayed for detection of ALP binding using 2.5 µl of 0.005 M para-nitrophenol phosphate (PNPP) in Tris or EPPS buffer at pH 7.5. The hydrolysis of the substrate results in yellow (PNPP) or blue-green (BCIP) color that is directly proportional to the amount of ALP bound to the bacteria. Alkaline phosphatase (ALP) activity was tested using common substrates such as BCIP (bromo-chloro-indolyl-phosphate) forming a blue/green color in Tris buffer, pH 7.5. After 10 minutes at room temperature the samples were read in a plate reader (Biotek Powerwave Absorbance Reader) at a wavelength of 405 nm. The parallel set of bacteria was run without addition of ALP as controls.

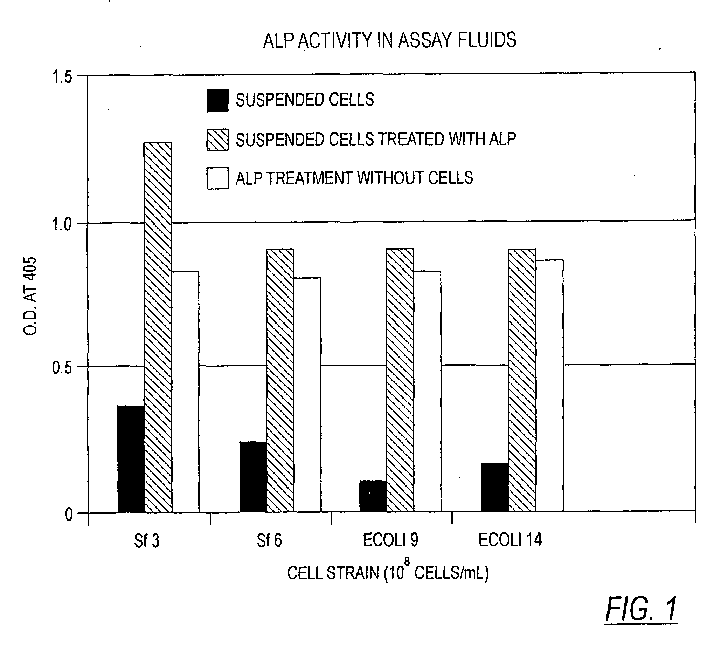

Intestinal ALP binding to bacteria cells was observed. In Figure 1, the striped bars show that suspended cells after ALP treatment and washings had more intestinal ALP activity than untreated cells (the solid bars). The solid bars do show that suspended cells not treated with intestinal ALP did have some ALP activity, believed to be from native ALP in the bacteria. As a control, the ALP activity of the treatment solutions show the maximum activity expected without contribution from native ALP.

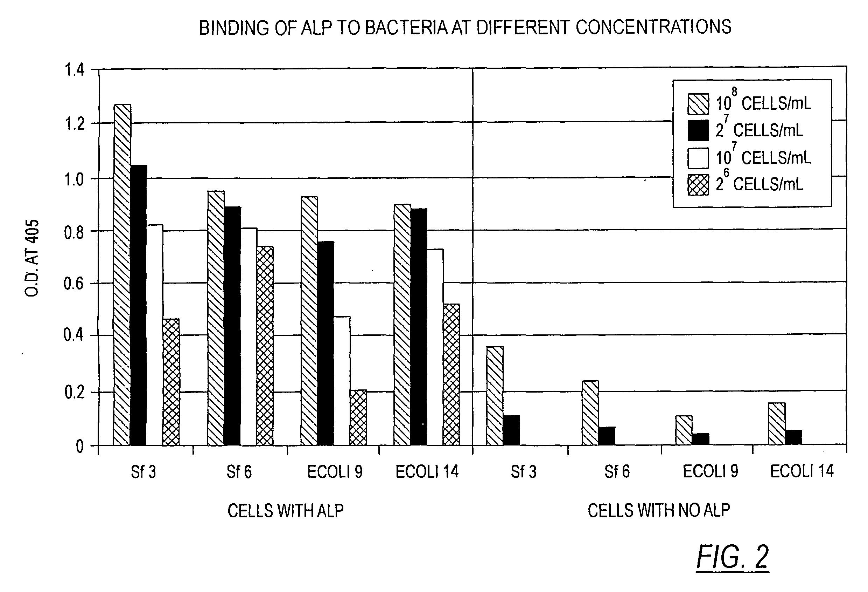

Figure 1 demonstrates intestinal ALP binding to all bacterial strains tested. Both gram positive bacteria such as *Staphylococcus aureus (Sf)* strains # 3 and #6 and gram negative bacteria such as *Escherichia Coli (E. Coli)* strains # 9 and 14 were found to bind the ALP. Again the striped bars being significantly larger than the solid bars demonstrate this. Figure 2 shows that the amount of ALP bound or activity generated is directly proportional to the amount of bacteria cells present. The ALP activity of the suspended cell increased with increasing amounts of cells.

The mechanism of the binding of ALP to the bacterial cells is not fully understood, but it is believed that glycated peptides in ALP or other glycoproteins are binding to the protein receptors anchored in the cell wall or are binding the peptidoglycon membrane. Both gram positive and gram negative bacteria are known to have protein receptors in their outer membranes. For gram negative bacteria, the outer lipopolysaccharide membrane has receptor proteins. For gram positive bacteria the outer peptidoglycon membrane has receptor proteins.

### Comparative Example 2

### Bacteria assay by binding of non-glycated protein to bacteria

As a control, an enzymatic protein lacking glycation, beta-galactosidase, was tested for binding to bacteria cell walls. The bacteria from both *Staph.* and *E. coli* were tested for beta-galactosidase binding. The beta-Galactosidases (20 mU) were added to saline suspensions of 10⁸ cells/ mL of both bacteria and were assayed as well as the pellets (cells re-suspended in water) and supernatants after spinning the bacteria using dimethylacridinium B-D-galactose (DMAG) as the substrate. The assay to determine the amount of enzyme was to add 10 µL of aqueous DMAG (0.5 mM) and 5 µL of aqueous tris buffer (1M) adjusted to pH 7.5 or test bacteria (10⁷ cells) and H₂O to 100 µl. Bright yellow color of DMAG changes to light green to dark blue in 5-30 minutes (with beta-galactosidase in 5 min) which is read at 634 nm on a plate reader.
Beta-D- galactosidase is a non-glycoprotein and non-membrane protein. In these experiments, beta-D- galactosidase did not bind bacteria and no measurement of bacteria was possible.

### Example 3

### Bacteria assay by binding of glycated proteins to bacteria

Bacterial cells (1 to 4.5 x 10⁷ cells/mL) were washed twice with water after centrifugation to separate cells into a packed pellet from the supernatant liquid. The washed cells in pellet form were suspended in 20 ul ofN-2-hydroxyethyl piperazine-N'-[3-propane sulfonic acid] EPPS buffer (50 mM at pH 8.0) and 30 µL of water. Glycated protein(s) (2-40 µg) were added. In some cases a glycated protein (2-40 µg) and bovine intestinal alkaline phosphatase (ALP) (2 µg or 10,000 Units) were added and the binding of the glycated protein measured by the reduction of binding of ALP.

The mixture of glycated protein and bacterial cells was left at 25° C for 15 minutes. The mixture was then centrifuged at 30,000 rpm for 30 minutes after which the bacterial cells formed a pellet at the bottom of the tube and were washed with water 4-5 times (50 µL). Centrifugation allows separation of glycoprotein bound to the bacteria cells from unbound glycated protein(s).

After washing, the bacterial pellets were suspended in 50 µL of borate buffer (25 mM at pH 9.0). A 5 µL aliquot of the suspension was assayed for detection of ALP binding by adding 5 µL of para-nitrophenol phosphate (PNPP, 100 mM), 50 µL sodium borate buffer ( 25 mM at pH 9.0) and 140 µL of water. The hydrolysis of the PNPP substrate resulted in a yellow color. The color is read at 405 nm using a ELISA plate reader between 15-30 min. The absorbance is directly proportional to the amount of ALP bound to the bacteria cell adhesions for glycated groups.

By comparison, various glycated and non-glycated proteins were tested for binding to bacteria (See Table 1). Albumin, prealbumin, alpha-1-antitrypsin, alpha-1-microglobulin, retinol binding protein, alpha-1-acid glycoprotein, alpha-2-glycoprotein, transferrin, Tamm-Horsfall glycoprotein and immunoglobulins were all known glycated proteins as received from suppliers. Hemoglobin, lysozyme, and myoglobin are all known non-glycated proteins as received from suppliers. All proteins were found to be binding the bacteria cell by measurements of bound protein using comassie brilliant blue.

Only a protein binding to the cell adhesions for glycated groups causes the inhibition of the binding of ALP by bacteria. A protein binding to the cell adhesions for glycated groups provides a positive number in Table 1. For example, albumin prevented 50% of ALP from binding to E. Coli bacteria. As seen in Table 1 all glycated proteins inhibited the binding of ALP by bacteria. Non-glycated proteins such as hemoglobin, myoglobin and lysozyme did not inhibit the binding of ALP. As a control, three non glycated polypeptides (polyarginine, polylysine, polyhistidine) were tested and not found to inhibit ALP activity.

**Table 1**

| **Demonstration of binding of glycated proteins to bacteria** | | |
|---|---|---|
| Added protein | *E.coli* | *S. faec.* |
| Albumin | 50% | 61% |
| Prealbumin | 50% | 57% |
| Tamm-Horsfall Glycoprotein | 40% | 49% |
| alpha-1-Antitrypsin | 84% | 74% |
| Myoglobin (non-glycated) | NI | NI |
| Hemoglogin (non-glycated) | NI | NI |
| Transferrin | 75% | 75% |
| Retinol Binding Protein | 81% | 83% |
| alpha-1-Acid glycoprotein | 86% | 90% |
| beta-2-Glycoprotein | 74% | 61% |
| Lysozyme (non-glycated) | NI | NI |
| IgG, IgA, IgM and Fragments | 63% | 71% |
| Polylysine, poly arginine poly histidine | NI | NI |

| | | |
|---|---|---|
| *NI= no inhibition | | |

ALP is an example of a glycated protein having enzymatic functionality and generating a signal, as demonstrated in Example 1. Other examples of enzymatic glycated proteins include acid phosphatase, fucosidase, phospholipase, glucocerebrosidase, hydrolase, arylsufatase A, amylases, cellobiohydrolase, and peroxidase.

Blocking additives can be used to block competing reactions and reduce interference or act as spreading agents. Examples are the non-binding glycoproteins of Example 3. Others are polymers such as poly (vinyl pyrrolidone) or polyvinyl alcohol and proteins such as casein, gelatin, albumin, hydrophobic cellulose, and polysaccharides.

### Example 4

### Bacteria assay by binding of ALP iso-forms to bacteria

Bacterial cells (1 to 4.5 x 10⁷ cells/mL) were washed twice with water after centrifugation to separate cells into a packed pellet from the supernatant liquid. The washed cells in pellet form were suspended in 20 µL of EPPS buffer (50 mM at pH 8.0) and 30 µL of water. Hemoglobin (20 µg) was added as a blocking additive. Alkaline phosphatase (ALP) (100 mUnits) from intestine, placenta, and bacteria sources were added.

The mixture of glycated protein and bacterial cells was left at 25° C for 15 minutes. The mixture was then centrifuged at 30,000 rpm for 30 minutes after which the bacterial cells formed a pellet at the bottom of the tube and were washed with water 4-5 times (50 µL). Centrifugation allows separation of glycoprotein bound to the bacteria cells from unbound glycated protein(s).

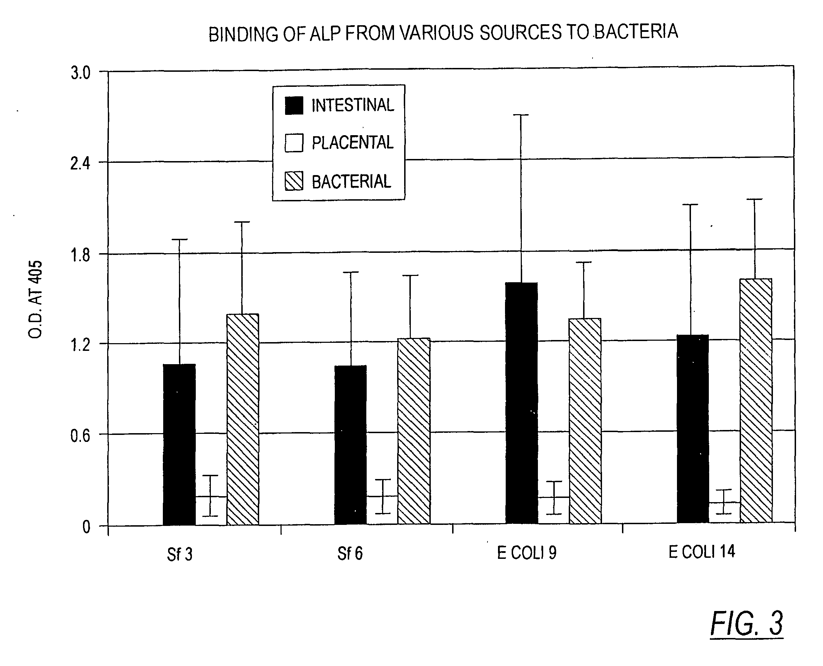

After washing, the bacterial pellets were suspended in 50 µL of sodium tetraborate buffer (25 mM at pH 9.5). A 5 µL aliquot of the suspension was assayed for detection of ALP binding by adding 5 µL of para-nitrophenol phosphate (PNPP, 100 mM), 50 µL sodium borate buffer ( 25 mM at pH 9.0) and 140 µL of water. The hydrolysis of the PNPP substrate resulted in a yellow color. The color is read at 405 nm using an ELISA plate reader between 15-30 min and the absorbance is directly proportional to the amount of ALP bound to the bacteria cell adhesions for glycated groups. The results are illustrated in Figure 3.

A comparison of ALP isozymes from placenta, bacterial and intestine sources allows an understanding of what glycosylation is needed for binding. The ISO forms of intestinal, liver, bone, and placental ALP have differences in carbohydrate structures and amount of sialic acid. Intestinal ALP lacks terminal sialic acids on its carbohydrate chains while placenta and bacterial have sialic acid residues. Bacterial ALP lacks a membrane binding glycophospholipid portion present in the mammalian ALP. Placenta ALP contains fucose, mannose and galactose while intestinal ALP has a high hexose and hexoamine content.

According to Figure 3, the glycophospholipids are not requirements for glycoprotein binding to bacteria as the bacterial ALP binds bacteria but lacks the glycophospholipid. All ALP bound to bacteria to some extent although placenta ALP exhibited the lowest enzyme activity as well as lowest binding to bacteria. This result supported our belief that certain degrees of glycosylation are better binders for bacteria.

Polylysine-conjugated intestinal ALP was also found to bind bacteria. The conjugation of ALP with a non-glycated peptide was not found to inhibit binding to bacteria and could provide linker arms for labels.

### Example 5

### Bacteria assay in presence of carbohydrates, polysaccharides, glycopeptides and lectins

Bacterial cells (1 to 4.5 x 10⁷ cells/mL) were washed twice with water after centrifugation to separate cells into a packed pellet from the supernatant liquid. The washed cells in pellet form were suspended in 20 µl of EPPS buffer (50 mM at pH 8.0) and 30 µL of water. Hemoglobin (20 µg) was added as a blocking additive. Alkaline phosphatase (ALP) (100 mUnits) from bovine intestine and 15 µg of simple carbohydrates or proteoglycan or lectins, were added.

The mixture of glycated protein and bacterial cells was left at 25° C for 15 minutes. The mixture was then centrifuged at 30,000 rpm for 30 minutes after which the bacterial cells formed a pellet at the bottom of the tube and were washed with water 4-5 times (50 µL). Centrifugation allows separation of glycoprotein bound to the bacteria cells from unbound glycated protein(s).

After washing, the bacterial pellets were suspended in 50 µL of sodium tetraborate buffer (25 mM at pH 9.5). A 5 uL aliquot of the suspension was assayed for detection of ALP binding by adding 5 µL of para-nitrophenol phosphate (PNPP, 100 mM), 50 µL sodium borate buffer ( 25 mM at pH 9.0) and 140 µL of water. The hydrolysis of the PNPP substrate resulted in a yellow color. The color is read at 405 nm using a ELISA plate reader between 15-30 min and the absorbance is directly proportional to the amount of ALP bound to the bacteria cell adhesions for glycated groups.

The binding of ALP to bacteria was shown by an absorbance of 1.8 to 2.0 in Table 2 in the absence of carbohydrates, proteoglycans, and lectins. The monosaccharides (simple carbohydrates) including Glucose, Mannose, Galactose and Sialic acid did not produce any effect on bacteria binding of ALP (all sources). Therefore simple carbohydrates are not involved in the binding and are not suitable as bacterial binders for attachment to detection labels. This also supports the need for glycopeptides or glycoproteins as binders rather than simple glyco-units.

Polysaccharides weakly inhibited the bacteria binding of ALP to a degree depending on the repeating carbohydrate unit. These results show that polysaccharides are involved in the binding of bacteria with ALP. Polysaccharides with N-acetylgalactosamine were more inhibitory and likely contained residual peptide units. By contrast lipopolysaccharide (LPS) was without any effect for the sources tested (B4 and B8 from 2 different serotypes of E.coli). Lipopolysaccharide contains Lipid A and O-antigen on the outer structure and does not expose its polysaccharide core.

Lipoteichoic acid is an example of polysaccharides with repeating carbohydrate and amino acid (Hyl) units. The structure of the polysaccharide varies with the source of LTA. Structures with and without N-acetylgalactosamine are known. In our results LTA (S. sanguis) strongly inhibits the bacteria bound ALP activity, whereas, depending on the source, varying or lack of inhibition was observed. Teichoic acid with repeating carbohydrate and amino acid (Hyl) units itself was found equally inhibitory. This supports our belief that the binding of glycopeptides to bacteria involves carbohydrate and amino acid components.

Lectins are proteins found in plant seeds which bind polysaccharides and monsaccharides attached to peptides. As seen in Table 2 lectins inhibited the bacteria binding of ALP depending on the polysaccharide unit that the lectin bound. These results also support the involvement of glycopeptides in the binding of bacteria and the ALP. The lectin binds the glyco group of ALP and prevents it from reacting with bacteria. Since several of the lectins are active but only bind one type of glyco group, several types of glyco peptide groups can cause binding of ALP to bacteria.

**Table 2**

| ***Additional carbohydrates, proteoglycan, and lectins*** | ***E.coli*** | ***S. faec.*** |
|---|---|---|
| None | 1.8 | 2.0 |
| | | |

| Simple carbohydrate | | |
|---|---|---|
| Glucose (β-D-Glucose) | 1.8 | 2.4 |
| Galactose (Gal or β-D-Galactose) | 2.0 | 2.0 |
| Fucose | 2.0 | 2.3 |
| Mannose (Man) | 1.7 | 2.4 |
| Sialic Acid (N-Acetyleneuaminic Acid) | 2.0 | 1.7 |
| Muramic Acid | 1.8 | 2.1 |
| GlcNAc (N-Acetyl-β-D-Glucosamine) | 2.0 | 2.0 |
| GalNAc (N-Acetyl-β-D-Galactosamine) | 1.8 | 1.9 |
| Glucuronic acid | 1.9 | 2.0 |
| Iduronic acid | 1.9 | 2.0 |
| | | |

| Polysacharide | | |
|---|---|---|
| Chondroitin sulfate A | 1.1 | 1.3 |
| (repeating GalNAc & glucuronic) | | |
| Chondroitin sulfate B | 0.9 | 0.5 |
| (repeating GalNAc & iduronic acid) | | |
| Hyaluronic Acid | 1.8 | 2.0 |
| (repeating GlcNAc & glucuronic acid) | | |
| Lipopolysaccharide | 1.8 | 2.0 |
| | | |

| Glycopeptide | | |
|---|---|---|
| Lipoteichoic acid | 0.2 | 0.2 |
| (from S. sanguis) | | |
| | | |

| Lectins that bind glycopeptides | | |
|---|---|---|
| Euonymus Europeus (Gal-Gal) | 1.6 | 1.8 |
| Bauhinia Purpurea (Gal-GalNAc) | 0.3 | 0.4 |
| Maackia Amurensis (Sialic Acid) | 0.1 | 0.1 |
| Concanavalin A (Man,Glc) | 0.0 | 0.1 |
| Caragana Arborescens (GalNAc) | 0.8 | 1.0 |

The data in Example 5 shows that the glycated portion can be a polysaccharides or a monosaccharide attached to at least one peptide. Examples of polysaccharides or monsacharides include those in Table 2.

### Example 6

### Alternative separation method of glycated proteins bound to bacteria

Bacteria bound to alkaline phosphatase (ALP) can be separated using a membrane (low protein binding Nylon 66 Loprodyne) on backed microtiter plates (Nunc Nalge International).

The loprodyne-membrane-backed plates were treated with 1 or 2% detergent (Tween 20 or TritonX305) in water or buffers (TBS: Tris, 25mM, pH 7.6 containing 150 mM NaCl or KCO3: 0.1M, pH 9.6) overnight at room temperature. Blocking solutions were vacuum filtered. Bacteria suspensions (10⁷ cells, 100 µl) in saline were combined with 50 µl EPPS buffer (0.05M, pH 8.1) and 50 µl H₂O containing 20 mU ALP. The combined solution was incubated for 15 min at 37° C on a shaker and then added to the loprodyne-membrane-backed plate.

The solution was vacuum filtered leaving bacteria adhered on the membrane and then washed twice with 2% Tween20 in water. To the washed membrane, 200 µl of H₂O with 50 µl Glycine (0.05M, pH 10.4) and containing 1mM PNPP were added and the color formed due to the bacteria bound ALP read at 405nm.

**Table 3**

| **Condition** | **Bacteria Binding to ALP (O.D. at 405nm)** | | |
|---|---|---|---|
| ALP concentration | 1.0 mU | 2.0 mU | 5.0 mU |
| No Bacteria | 0.04 | 0.05 | 0.16 |
| Plus Bacteria | 0.13 | 0.30 | 0.83 |

The separation of E. Coli with bound ALP from unbound ALP is shown by a size exclusion membrane in Example 6 and by centrifugation in Examples 1-5. The size of E. Coli is 1 x 1 x 2 µm and any membrane, filter or device trapping particles of this size would be acceptable. These include microfluidic devices, filters, column chromatography and chromatography strips. The mass of E. Coli is 1.6 x 10⁻¹² gm/cell and any membrane, filter or device trapping a mass of this size would be also acceptable.

### Example 7

### Effect of Divalent Cations in Protein Binding to Bacteria

Bacterial cells (1 to 4.5 x 10⁷ cells/mL) were washed twice with water after centrifugation to separate cells into a packed pellet from the supernatant liquid. The washed cells in pellet form were suspended in 20 µL of EPPS buffer (50 mM at pH 8.0) and 30 µL of water. Bovine intestinal alkaline phosphatase (ALP) (2 µg or 10,000 Units) was added and 0.2 mM of several cations.

The mixture of glycated protein and bacterial cells was left at 25° C for 15 minutes. The mixture was then centrifuged at 30,000 rpm for 30 minutes after which the bacterial cells formed a pellet at the bottom of the tube and were washed with water 4-5 times (50 µL). Centrifugation allows separation of glycoprotein bound to the bacteria cells from unbound glycated protein(s).

After washing, the bacterial pellets were suspended in 50 µL of borate buffer (25 mM at pH 9.0). A 5 µL aliquot of the suspension was assayed for detection of ALP binding by adding 5 µL of para-nitrophenol phosphate (PNPP, 100 mM), 50 µL sodium borate buffer (25 mM at pH 9.0) and 140 µL of water. The hydrolysis of the PNPP substrate resulted in a yellow color. The color was read at 405 nm using an ELISA plate reader between 15-30 min; the absorbance is directly proportional to the amount of ALP bound to the bacteria cell.

**Table 4**

| **Condition** | **O.D. at 405 nm** | |
|---|---|---|
| | No ALP | With ALP |
| **No cation** | **0.18** | **0.30** |
| + CaCl₂ (1 mM) | 0.23 | 0.36 |
| + MgCl₂ (1 mM | 0.20 | 0.44 |
| **+ ZnCl₂ (0.2 mM)** | **0.23** | **0.53** |

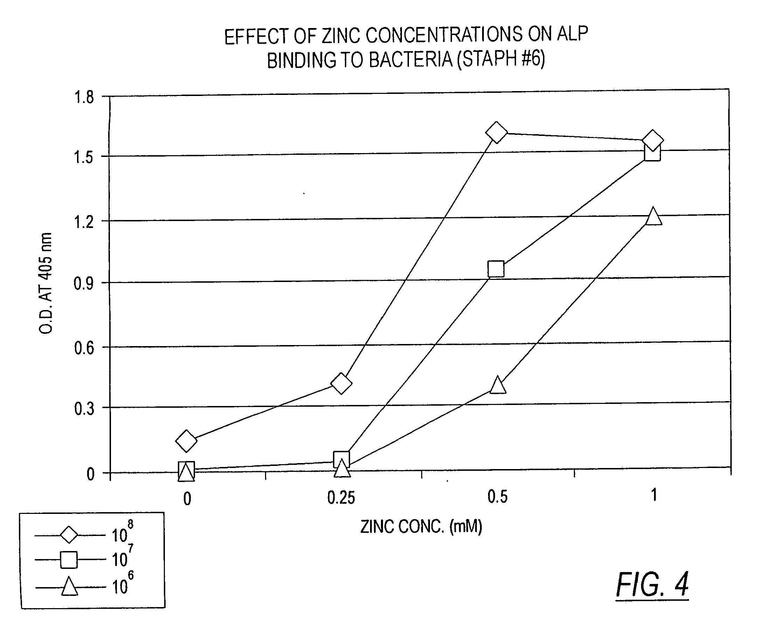

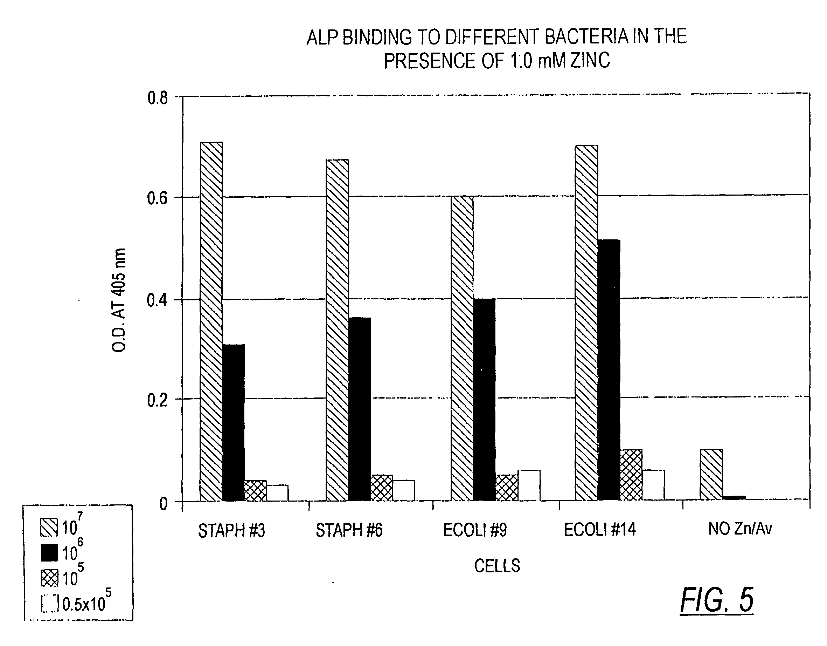

As seen in Table 4, Zn²⁺ (0.2 mM) resulted in significantly higher binding of ALP to the bacteria. Concentration dependent binding study has been performed in the presence of increasing concentration of zinc with S. faecalis strain as shown in Figure 4. Result indicated that optimum binding occurs at 1 mM zinc concentration. The studies have been continued with different strains of bacteria in the presence of 1 mM Zn²⁺ and the ALP binding could be monitored even at 5x10⁶ bacteria concentration (Fig.5).

### Example 8

### Effect of Various Cation on ALP Binding to Bacteria

Bacterial cells (1 to 4.5 x 10⁷ cells/mL) were washed twice with water after centrifugation to separate cells into a packed pellet from the supernatant liquid. The washed cells in pellet form were suspended in 20 µL of EPPS buffer (50 mM at pH 8.0) and 30 µL of water. Bovine intestinal alkaline phosphatase (ALP) (2 µg or 10,000 Units) was added and 0.2 mM of each cation.

The mixture of glycated protein and bacterial cells was left at 25° C for 15 minutes. The mixture was then centrifuged at 30,000 rpm for 30 minutes after which the bacterial cells formed a pellet at the bottom of the tube and was washed with water 4-5 times (50 µL). Centrifugation allows separation of glycoprotein bound to the bacteria cells from unbound glycated protein(s).

After washing, the bacterial pellets were suspended in 50 µL of borate buffer (25 mM at pH 9.0). A 5 µL aliquot of the suspension was assayed for detection of ALP binding by adding 5 µL of para-nitrophenol phosphate (PNPP, 100 mM), 50 µL sodium borate buffer (25 mM at pH 9.0) and 140 µL of water. The hydrolysis of the PNPP substrate resulted in a yellow color. The color was read at 405 nm using an ELISA plate reader between 15-30 min; the absorbance is directly proportional to the amount of ALP bound to the bacteria cell.

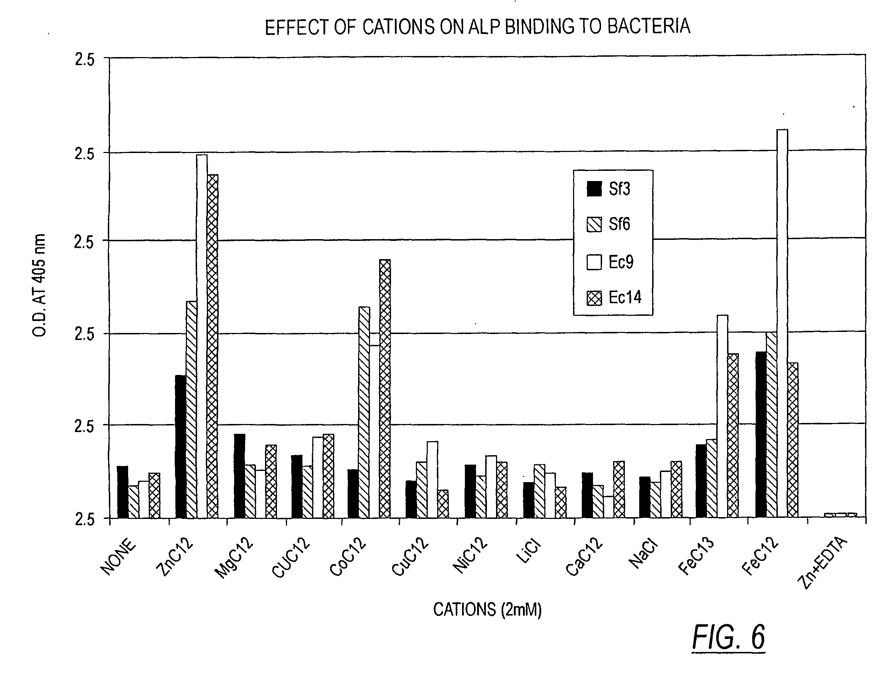

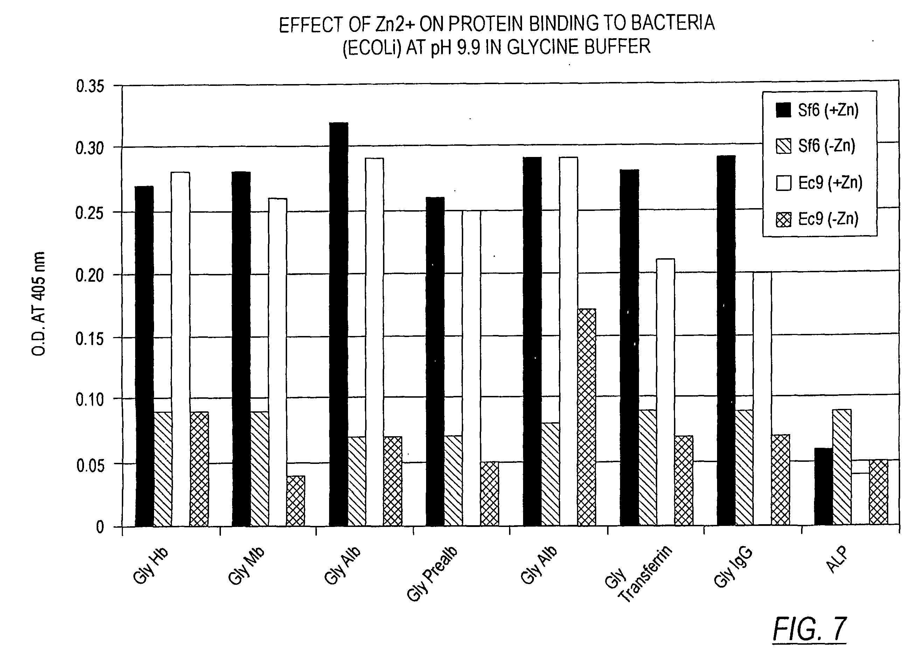

Zinc dependency of all the protein binding to bacterial cell wall had been observed as mentioned before. The effect of various cations (2 mM) on the binding of bovine intestinal mucosa ALP (Biozyme) to different bacteria is shown in Figure 6. In addition to zinc, Cu²⁺, Fe²⁺ and Fe³⁺ also seem to stimulate ALP- binding in both Gram-positive and Gram-positive strains of bacteria (Sf- Staph. faec.; Ec: E. coli). Figure 6 also indicates total inhibition of ALP activity in the presence of EDTA (10 mM). Zinc had been used for continuing ALP binding studies. Alkaline phosphatase binding to bacteria seems to be very dependent on the presence of cations as seen in Figure 6. The data in Figure 7 shows the binding of various glycated proteins in absence and presence of zinc which clearly demonstrates cation dependency of all the proteins tested for binding to both Gram-positive and Gram-negative bacterial cell wall. The amount of ALP used for this study was so small it can only be detected by measuring enzymatic activity.

### Example 9

### Optimum Conditions for Concentration of Zn in ALP Binding

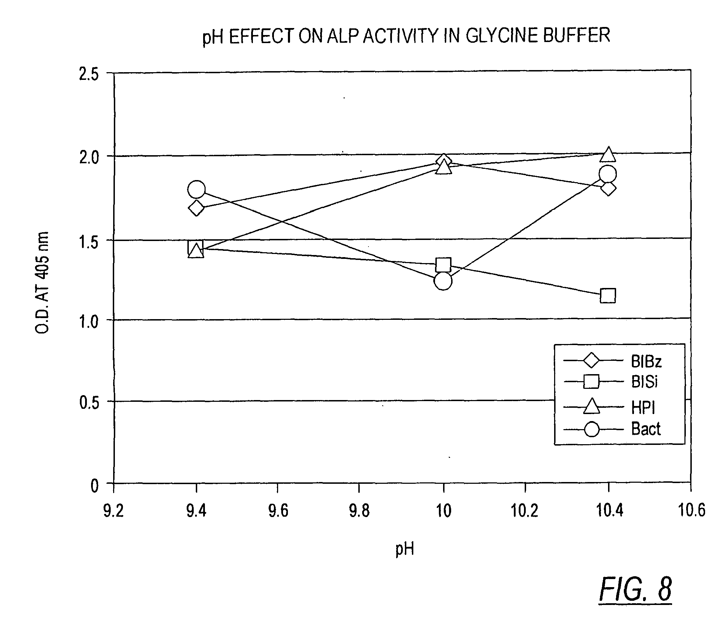

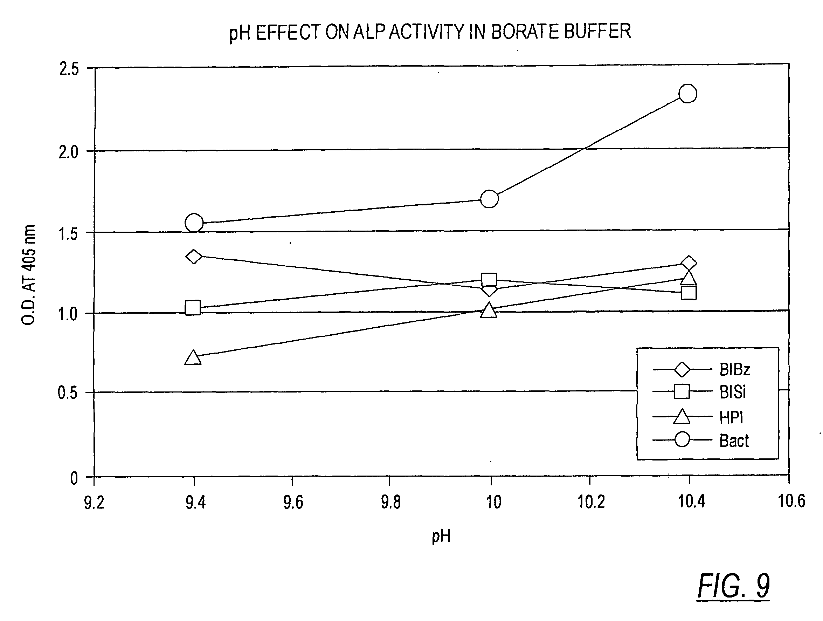

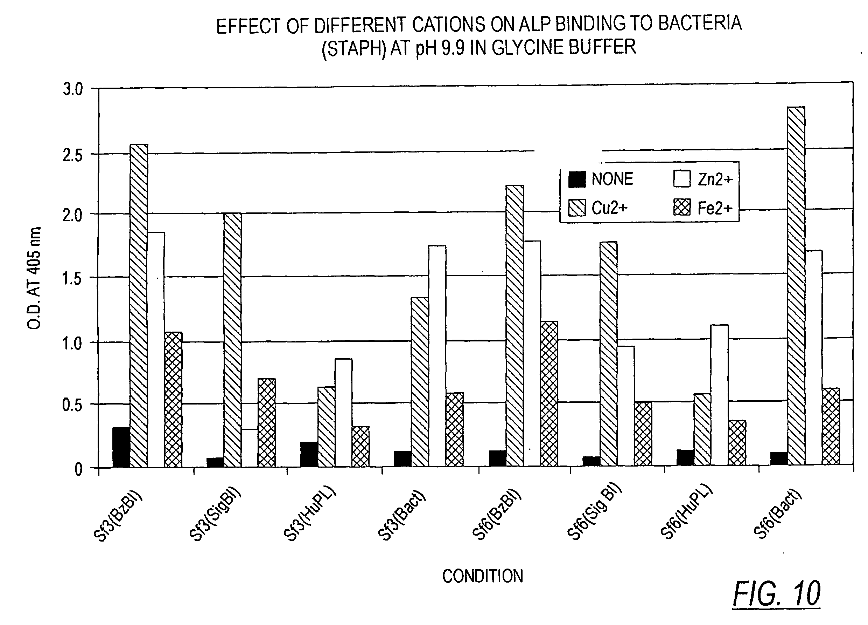

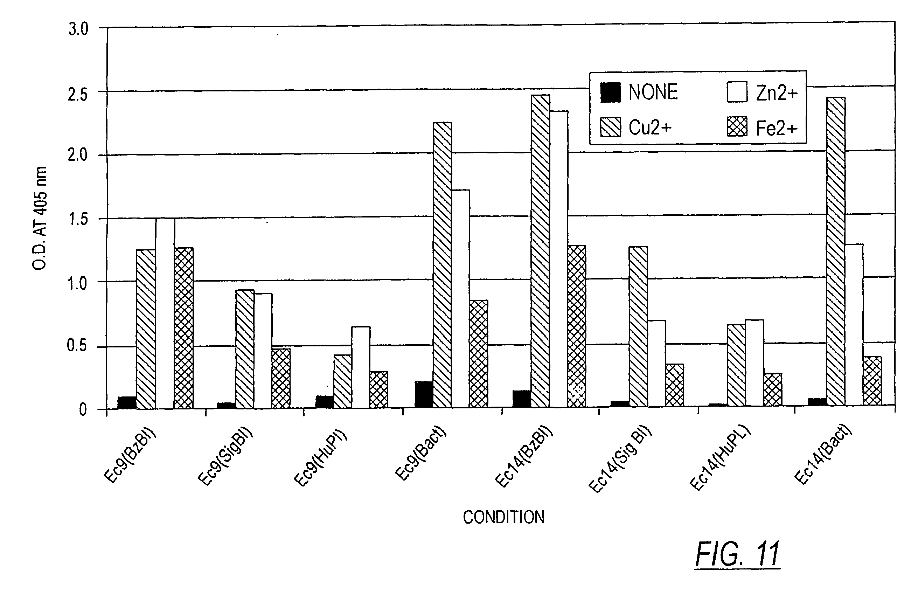

The human placental ALP activity is comparable to ALP from other sources when assayed in glycine buffer as seen in Figures 8-9. Among three cations effective for binding of ALP to the bacteria, zinc was the best metal when the ALP-bound bacteria (both Staph and E.coli) were assayed in glycine buffer, pH 10.0 (Figs. 10-11). The binding was conducted at pH 8.0 in EPPS buffer.

In Figures 8-11, the following abbreviations are used:
B1BZ = bovine intestinal from a first vendor
B1Si = bovine intestinal from a second vendor
HPL = human placenta
Bact = bacterial

## Claims

1. A method for measuring the bacteria content of fluids comprising:
a. binding an effective amount of a glycoprotein with bacteria contained in a sample of fluid, said glycoprotein having an enzymatic functionality generating a signal to indicate its presence;
b. separating excess unbound glycoprotein from said fluid sample after reacting said glycoprotein with bacteria in said sample in step (a);
c. measuring the amount of said label remaining after separating said excess unbound glycoprotein of (b); and
d. determining the bacteria content of said sample as related to the amount of said label measured in step (c);
wherein said glycoprotein is at least one member of the group consisting of intracellular enzymes and said enzymes inherently provide a label moiety serving as said label by directly binding said bacteria.

2. A method of Claim 1 wherein said glycoprotein has a binding constant to bacteria of at least 10⁶ and at least 100 binding sites.

3. A method of Claim 1 wherein said intra cellular enzymes are selected from the group consisting of glutamate hydrogenase, ALP, and lactate dehydrogenase.

4. A method of Claim 1 wherein said glycoprotein is an intracellular enzyme selected from the group consisting of alkaline phosphatase, acid phosphatase, fucosidase, mannosidase, hexaminidase, alpha-galactosidase, phospholipase, hyaluronidase, glucocerebrosidase, hydrolase, arylsulfatase A, amylases, cellobiohydrolase, and peroxidase.

5. A method of Claim 3 wherein said enzyme is alkaline phosphatase (ALP).

6. A method of Claim 5 wherein said ALP is intestinal ALP.

7. A method of Claim 5 wherein ALP is measured by adding as a reagent PNPP.

8. A method of Claim 7 wherein the color developed by said reagent is read at a wavelength of 405nm.

9. A method of Claim 1 further comprising adding to said sample blocking compounds selected from the group consisting of polymers, non-glycated proteins, non-glycated polypeptides, and polysaccharides.

10. A method of Claim 1 further comprising adding at least one cation capable of increasing the binding of said glycoprotein to bacteria.

11. A method of Claim 10 wherein said cation is at least one member of the group consisting of zinc, copper and iron.

12. A method of Claim 11 wherein said cation is zinc.

## Patentansprüche

1. Verfahren zur Messung des Bakteriengehalts von Flüssigkeiten, das Folgendes beinhaltet:
a. Bindung einer wirksamen Menge eines Glykoproteins an Bakterien, die in einer Flüssigkeitsprobe enthalten sind, wobei besagtes Glykoprotein eine enzymatische Funktionalität besitzt, die ein Signal erzeugt, das sein Vorhandensein anzeigt;
b. Trennung von überschüssigem ungebundenem Glykoprotein von besagter Flüssigkeitsprobe nach der Reaktion von besagtem Glykoprotein mit Bakterien in besagter Probe in Schritt (a);
c. Messung der Menge besagten Labels, die nach der in Schritt (b) erfolgten Trennung von überschüssigem ungebundenem Glykoprotein zurückbleibt, und
d. Bestimmung des Bakteriengehalts besagter Probe in Relation zu der in Schritt (c) gemessenen Menge besagten Labels;
wobei besagtes Glykoprotein zumindest ein Mitglied der aus intrazellulären Enzymen bestehenden Gruppe ist und besagte Enzyme ihrer Natur nach einen Label-Anteil bieten, der als besagtes Label dient, indem es besagte Bakterien direkt bindet.

2. Verfahren nach Anspruch 1, bei dem besagtes Glykoprotein eine Bindungskonstante für Bakterien von mindestens 10⁶ und mindestens 100 Bindungsstellen hat.

3. Verfahren nach Anspruch 1, bei dem besagte intrazelluläre Enzyme aus der Gruppe ausgewählt werden, die aus Glutamathydrogenase, ALP und Laktatdehydrogenase besteht.

4. Verfahren nach Anspruch 1, bei dem besagtes Glykoprotein ein intrazelluläres Enzym ist, ausgewählt aus der Gruppe, die aus alkalischer Phosphatase, saurer Phosphatase, Fukosidase, Mannosidase, Hexaminidase, Alpha-Galaktosidase, Phospholipase, Hyaluronidase, Glukocerebrosidase, Hydrolase, Arylsulfatase A, Amylasen, Cellobiohydrolase und Peroxidase, besteht.

5. Verfahren nach Anspruch 3, bei dem besagtes Enzym alkalische Phosphatase (ALP) ist.

6. Verfahren nach Anspruch 5, bei dem besagte ALP intestinale ALP ist.

7. Verfahren nach Anspruch 5, bei dem ALP durch Hinzufügen von pNPP als Reagens gemessen wird.

8. Verfahren nach Anspruch 7, bei dem die Wellenlänge der durch besagtes Reagens entwickelten Farbe 405nm beträgt.

9. Verfahren nach Anspruch 1, das ferner das Hinzufügen von Blockerverbindungen aus der Gruppe, die aus Polymeren, nicht glykierten Proteinen, nicht glykierten Polypeptiden und Polysacchariden besteht, zu besagten Proben umfasst.

10. Verfahren nach Anspruch 1, das ferner das Hinzufügen von mindestens einem Kation umfasst, das die Fähigkeit besitzt, die Bindung von besagtem Glykoprotein an Bakterien zu verbessern.

11. Verfahren nach Anspruch 10, bei dem besagtes Kation mindestens ein Mitglied der Gruppe ist, die aus Zink, Kupfer und Eisen besteht.

12. Verfahren nach Anspruch 11, bei dem besagtes Kation Zink ist.

## Revendications

1. Procédé de mesure de la teneur en bactéries de fluides, dans lequel :
a) on fixe une quantité efficace d'une glycoprotéine par des bactéries contenues dans un échantillon de fluide, la glycoprotéine ayant une fonctionnalité enzymatique produisant un signal pour indiquer sa présence ;
b) on sépare de la glycoprotéine non fixée en excès de l'échantillon de fluide après réaction de la glycoprotéine sur des bactéries de l'échantillon au stade ( a ) ;
c) on mesure la quantité du marqueur restant après séparation de la glycoprotéine non fixée en excès de ( b ) ; et
d) on détermine la teneur en bactéries de l'échantillon comme étant reliée à la quantité du marqueur mesurée au stade ( c ) ;
dans lequel la glycoprotéine est au moins un élément du groupe consistant en des enzymes intracellulaires et ces enzymes procurent de manière inhérente un radical de marqueur servant de marqueur en fixant directement les bactéries.

2. Procédé suivant la revendication 1 dans lequel la glycoprotéine a une constante de fixation à des bactéries d'au moins 106 et d'au moins 100 sites de fixation.

3. Procédé suivant la revendication 1 dans lequel les enzymes intracellulaires sont choisis dans le groupe consistant en la glutamate hydrogénase, l'ALP, et la lactate déhydrogénase.

4. Procédé suivant la revendication 1 dans lequel la glycoprotéine est un enzyme intracellulaire choisi dans le groupe consistant en une phosphatase alcaline, en une phosphatase acide, en la fucosidase, en la mannosidase, en l'hexaminidase, en l'alpha-galactosidase, en une phospholipase, en l'hyaluronidase, en la glucocérébrosidase, en une hydrolase, en l'arylsulfatase A, en des amylases, en la cellobiohydrolase, et en une peroxydase.

5. Procédé suivant la revendication 3 dans lequel l'enzyme est une phosphatase alcaline ( ALP ).

6. Procédé suivant la revendication 5 dans lequel l'ALP est une ALP intestinale.

7. Procédé suivant la revendication 5 dans lequel on mesure l'ALP en l'ajoutant comme réactif PNPP.

8. Procédé suivant la revendication 7 dans lequel la couleur développée par le réactif est lue à une longueur d'onde de 405 nm.

9. Procédé suivant la revendication 1 comprenant, en outre, l'addition à l'échantillon de composés de blocage choisis dans le groupe consistant en des polymères, des protéines non-glycatés, des polypeptides non-glycatés et des polysaccharides.

10. Procédé suivant la revendication 1 comprenant, en outre, l'addition d'au moins un cation apte à augmenter la fixation de la glycoprotéine à des bactéries.

11. Procédé suivant la revendication 10 dans lequel le cation est au moins un élément du groupe consistant en le zinc, le cuivre et le fer.

12. Procédé suivant la revendication 11 dans lequel le cation est le zinc.
